(19) Europäisches Patentamt
European Patent Office
Office européen des brevets

(11) EP 4 796 214 A1

(12) EUROPEAN PATENT APPLICATION
published in accordance with Art. 153(4) EPC

(43) Date of publication:
**26.08.2026 Bulletin 2026/35**

(21) Application number: **24878822.6**

(22) Date of filing: **26.09.2024**

(51) International Patent Classification (IPC):
***B01J 23/44*** (2006.01) ***B01J 35/53*** (2024.01)
***B01J 32/00*** (2006.01) ***C07C 5/05*** (2006.01)
***C07C 5/09*** (2006.01) ***C07C 11/04*** (2006.01)
***C07C 11/06*** (2006.01)

(52) Cooperative Patent Classification (CPC):
**B01J 21/00; B01J 23/44; B01J 23/50; B01J 23/60; B01J 23/63; B01J 35/30; B01J 35/45; B01J 35/53; B01J 35/64; B01J 37/02; B01J 37/10; C07C 5/05; C07C 5/09; C07C 11/04; C07C 11/06**

(86) International application number:
**PCT/CN2024/121420**

(87) International publication number:
**WO 2025/082175 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.10.2023 CN 202311359592**

(71) Applicants:
- **China Petroleum & Chemical Corporation**
  **Beijing 100728 (CN)**
- **Sinopec (Beijing) Research Institute of Chemical Industry Co., Ltd.**
  **Beijing 100013 (CN)**

(72) Inventors:
- **YANG, Chenxi**
  **Beijing 100013 (CN)**
- **YUE, Yi**
  **Beijing 100013 (CN)**
- **PENG, Hui**
  **Beijing 100013 (CN)**
- **WANG, Qianyue**
  **Beijing 100013 (CN)**
- **MAO, Zuwang**
  **Beijing 100013 (CN)**
- **YI, Shuisheng**
  **Beijing 100013 (CN)**
- **GUO, Zhaojing**
  **Beijing 100013 (CN)**
- **TIE, Kai**
  **Beijing 100013 (CN)**

(74) Representative: **karo IP Patentanwälte PartG mbB**
**Steinstraße 16-18**
**40212 Düsseldorf (DE)**

# (54) EGGSHELL-TYPE CATALYST, MODIFIED CARRIER, AND PREPARATION METHOD THEREFOR AND USE THEREOF

(57) The present invention relates to an ultrathin eggshell-type highly dispersed selective hydrogenation catalyst, a modified carrier and a preparation method therefor and use thereof. The eggshell-type catalyst comprises a carrier and an active metal element. 95 wt% to 99 wt% of the active metal element relative to the total weight of the active metal element are distributed in the shell of the carrier with a thickness of 60 μm or less, calculated from an outer surface of the carrier as a starting point toward a center, and exhibit an eggshell-type distribution; and the active metal element has a dispersity of 50% to 80%. The active metal in the catalyst of the present invention has a highly dispersed state while being distributed in an extremely thin shell layer region on the carrier surface and exhibits excellent properties in the selective hydrogenation catalytic reaction.

EP 4 796 214 A1

25µm

Fig. 1

## Description

### TECHNICAL FIELD

The present invention belongs to the field of catalysts, and in particular relates to an ultrathin eggshell-type highly dispersed selective hydrogenation catalyst, a modified carrier, and a preparation method therefor and a use thereof.

### BACKGROUND TECHNOLOGY

Supported catalysts with metals as active components have wide applications in the petrochemical industry. They can be generally categorized into four types: uniform type, eggshell type, yolk type, and eggwhite type according to the differences in the active component enrichment sites. For precious metal supported catalysts, since they have scarce resources and high prices, an appropriate method needs to be adopted to enrich the precious metals on the carrier surface to form eggshell-type catalysts, thereby improving the utilization rate of the precious metals. Moreover, a thinner shell layer is beneficial to rapid surface reactions and diffusion-controlled catalytic reactions. However, it is usually considered that active metals very easily agglomerate or aggregate during the preparation of eggshell-type catalysts, leading to a decreased dispersity and a decreased utilization rate and a poor catalytic performance of the metals. Therefore, how to control the enrichment of active metals on the thin shell layer of the carrier while maintaining their uniform distribution under the premise of keeping the total amount unchanged is a hotspot and a difficult point of the current research.

CN101730588A discloses a catalyst for the selective hydrogenation of alkynes and a method for producing said catalyst. An impregnating solution is obtained by dissolving an active metal compound and a co-catalyst metal compound in a mixed solution of water and an organic solvent, then a support is impregnated with the impregnating solution, and the impregnated support is calcined at last, thereby obtaining a catalyst with at least 90% of active components present in the support which has a thickness of not more than 250 μm.

CN103769094A discloses an eggshell-type catalyst for a selective hydrogenation reaction. The carrier is respectively impregnated in an aqueous solution of a reducing agent, an additive and an active component, and after drying, calcination and reduction, an eggshell-type catalyst with a low precious metal loading and a low cost is obtained.

CN111229216A discloses an eggshell-type silver catalyst and the preparation method and application thereof. An eggshell-type silver catalyst is obtained by first impregnating with an active precursor solution followed by an in-situ treatment via a chemical energy transfer-deposition method. The catalyst is suitable for the oxidative dehydrogenation of alcohols to produce aldehydes and the epoxidation reactions, in particular the reactions of catalyzing the oxidative dehydrogenation of isopentenol to produce isopentenal.

Current technical solutions (for example CN101730588A, CN103769094A, CN111229216A mentioned above) mostly use traditional impregnation methods, in which influencing factors (for example pH value, metal ion concentration, viscosity, drying process, etc.) are numerous and complex, making it difficult to regulate parameters such as the catalyst shell layer thickness, the active metal loading and the dispersity. Meanwhile, such methods often use various organic reducing agents, dispersants and solvents, which have low environmental friendliness and are difficult to enlarge production.

### CONTENTS OF THE INVENTION

To solve the problems existing in the prior art, the present invention provides an eggshell-type catalyst, in particular an ultrathin eggshell-type highly dispersed catalyst, a modified carrier, and a preparation method therefor and a use thereof. The method for preparing the modified carrier starts from regulating the surface properties of the carrier, treats a carrier precursor mixture of a carbon-containing compound by means of heat treatment, crystallization and irradiation, etc., enriches and increases the type and the density of oxygen-containing groups on the surface of the carrier. The catalyst prepared from the modified carrier can make the active metal have a highly dispersed state while being distributed in an extremely thin shell layer region on the carrier surface, and exhibit excellent properties in the selective hydrogenation catalytic reaction.

A first aspect of the present invention is to provide an eggshell-type, particularly ultrathin eggshell-type, highly dispersed catalyst, comprising a carrier and an active metal element. 95 wt% to 99 wt% of the active metal element relative to the total weight of the active metal element are distributed in the shell of the carrier with a thickness of 60 μm or less, calculated from an outer surface of the carrier as a starting point toward a center, and exhibit an eggshell-type distribution; the active metal element has a dispersity of 50% to 80%.

In one embodiment of the present invention, the active metal element comprises a main metal element and optionally an auxiliary metal element.

In one embodiment of the present invention, 95 wt% to 99 wt% of the active metal element relative to the total weight of the active metal element are distributed in the shell of the carrier with a thickness of 50 μm or less, calculated from

the outer surface of the carrier as a starting point toward the center; and/or

In one embodiment of the present invention, the active metal element has a dispersity of 60% to 80%, preferably greater than 70%, more preferably 71% to 80%, such as 72%, 73%, 74%, 75%, 76%, 77%, 78%, 79%; and/or

In one embodiment of the present invention, the active metal has an average particle size of less than 4 nm, preferably of 2-3.7 nm; and/or

In one embodiment of the present invention, the catalyst has a specific surface area of 20-150 $m^2/g$.

According to the present invention, the ratio of the weight of the main metal element to the weight of the carrier can be selected within a wide range. In a preferred embodiment of the present invention, the ratio of the weight of the main metal element to the weight of the carrier is (0.01-0.5):100, preferably (0.05-0.3):100, based on the weight of the main metal element.

According to the present invention, the main metal element can be selected within a wide range. In a preferred embodiment of the present invention, the main metal element is selected from at least one of the metal elements of Group VIIIB, preferably palladium.

According to the present invention, the ratio of the total weight of the auxiliary metal element to the weight of the carrier can be selected within a wide range. In a preferred embodiment of the present invention, the ratio of the total weight of the auxiliary metal element to the weight of the carrier is (0-5): 100, based on the weight of the auxiliary metal element.

According to the present invention, the auxiliary metal element can be selected within a wide range. In a preferred embodiment of the present invention, the auxiliary metal element is selected from at least one of metal elements of Group IB, Group IIB, Group IIIA, Group IIIA and Lanthanide, preferably at least one of silver, indium, gallium, cerium and zinc.

A second aspect of the present invention provides a method for preparing the catalyst according to the first aspect, comprising:

impregnating a modified carrier with a solution containing a source of an active metal element followed by activation to obtain the catalyst, a surface of the modified carrier having an oxygen-containing group with a density of 0.1-1.2 $mmol/m^2$, preferably 0.1-0.9 $mmol/m^2$, more preferably 0.15-0.9 $mmol/m^2$, and still more preferably 0.18-0.85 $mmol/m^2$.

In one embodiment, the modified carrier is at least one of alumina, alumina, magnesia, calcium oxide, hydrotalcite, silica, titania, ceria, preferably alumina.

Preferably, the oxygen-containing group is at least one of hydroxyl, carboxyl and a lactone group, and more preferably, the oxygen-containing group is hydroxyl, carboxyl and a lactone group. Preferably, a content of hydroxyl, carboxyl and the lactone group is:

the content of the hydroxyl is 4-40 mmol/g, preferably 6-30 mmol/g; and/or
the content of the carboxyl is 2.5-30 mmol/g, preferably 6-20 mmol/g; and/or
the content of the lactone group is 0.05-20 mmol/g, preferably 1.5-20 mmol/g.

In one embodiment, the modified carrier has a specific surface area of 20-150 $m^2/g$, preferably 30-130 $m^2/g$.

The activation step herein can be carried out in any suitable sequence.

In one embodiment of the present invention, the source of the active metal element comprises a source of the main metal element and optionally a source of the auxiliary metal element.

According to the present invention, the source of the main metal element can be selected within a broad range. In a preferred embodiment of the present invention, the source of the main metal element is selected from at least one of soluble compounds containing the main metal element (e.g., metal elements of Group VIIIB), preferably at least one of water-soluble palladium compounds, more preferably at least one of palladium nitrate, palladium chloride, sodium chloropalladate, palladium oxalate, palladium citrate.

Preferably, the ratio of the content of the source of the main metal element (e.g., a palladium compound), calculated as a metal element, to the weight of the carrier is (0.01-0.5):100, preferably (0.02-0.45):100.

According to the present invention, the source of the auxiliary metal element can be selected within a wide range. In a preferred embodiment of the present invention, the source of the auxiliary metal element is selected from at least one of soluble compounds comprising an auxiliary metal element (e.g. metal elements of Group IB, Group IIB, Group IIIA, Group IIIA and Lanthanide), preferably water-soluble salt compounds of the auxiliary metal element. By way of example, the compound of the auxiliary metal is at least one of soluble salt compounds of silver, indium, gallium, cerium and zinc. The ratio of the content of the source of the auxiliary metal element (the compound of the auxiliary metal), calculated as a metal element, to the weight of the carrier may be (0-5):100, preferably (0.02-4):100.

In one embodiment of the present invention, the activation method comprises a calcination stage, or an irradiation stage, or both of the calcination stage and the irradiation stage carried out in any sequence.

Preferably, a condition of the calcination comprises:

a temperature of 150-750 °C, and/or
a duration of 2-20 h, e.g., 4-20 h, and/or

an atmosphere of at least one of air, nitrogen, hydrogen, argon.

The calcination condition is more preferably a temperature of 180-725 °C, preferably 400-650 °C; a duration of 2-18 h, preferably 6-18 h; and an atmosphere of at least one of air, nitrogen, hydrogen and argon, preferably at least one of air, nitrogen and hydrogen.

In one embodiment of the present invention, the irradiation condition comprises: a ray source selected from a y ray, and/or an irradiation dose of 1-20 kGy, preferably 4-18 kGy.

In one embodiment of the present invention, the impregnation solution as used is an aqueous solution of a palladium compound or a compound of an auxiliary metal, or a mixture of the aqueous solutions of the above two compounds.

According to the present invention, when two or more impregnation solutions are used, the impregnation may be either a sequential impregnation or a co-impregnation. In the case of the sequential impregnation, each impregnation needs to be followed by drying prior to next impregnation. Calcination and activation may be carried out in steps, or may be carried out once after all components are loaded.

According to the present invention, the impregnation may be carried out once or multiple times. The activation is optionally carried out before impregnation, after impregnation, or at intervals between multiple times of impregnation. Preferably, when the activation comprises multiple stages, some stages of the activation are optionally conducted before impregnation, after impregnation, or at intervals between multiple times of impregnation.

A third aspect of the present invention is to provide a modified carrier for the eggshell-type catalyst, particularly an ultrathin eggshell-type highly dispersed catalyst. The surface of the modified carrier has an oxygen-containing group with a density of 0.1-1.2 mmol/m$^2$, preferably 0.1-0.9 mmol/m$^2$, more preferably 0.15-0.9 mmol/m$^2$, and still more preferably 0.18-0.85 mmol/m$^2$.

In one embodiment, the modified carrier is at least one of alumina, alumina, magnesia, calcium oxide, hydrotalcite, silica, titania, ceria, preferably alumina.

Preferably, the oxygen-containing group is at least one of hydroxyl, carboxyl and a lactone group, and more preferably, the oxygen-containing group is hydroxyl, carboxyl, and a lactone group. Preferably, a content of hydroxyl, carboxyl, and the lactone group is:

the content of the hydroxyl is 4-40 mmol/g, preferably 6-30 mmol/g; and/or
the content of the carboxyl is 2.5-30 mmol/g, preferably 6-20 mmol/g; and/or
the content of the lactone group is 0.05-20 mmol/g, preferably 1.5-20 mmol/g.

In one embodiment, the modified carrier has a specific surface area of 20-150 m$^2$/g, preferably 30-130 m$^2$/g.

A fourth aspect of the present invention is to provide a preparation method for the modified carrier, comprising mixing a dry material including a carrier precursor, a molding pore-expanding agent and an inorganic carbon with a liquid, followed by kneading, granulation and a surface modification to obtain the modified carrier, wherein the surface modification involves at least two methods of heat treatment, crystallization and irradiation.

Preferably, the inorganic carbon is selected from at least one of wood charcoal, bamboo charcoal, coconut shell charcoal, graphene, graphyne, and diamond.

The sequence of heat treatment, crystallization and irradiation during the surface modification can be selected arbitrarily according to practical reactions. In one embodiment, the surface modification involves crystallization and heat treatment, irradiation and heat treatment, or irradiation and crystallization, preferably crystallization and heat treatment, irradiation and heat treatment, wherein the operation sequence can be arbitrarily selected according to practical reactions, for example carrying out irradiation before heat treatment, or carrying out crystallization before heat treatment.

In one embodiment, the heat treatment condition includes a treatment with a heat treatment atmosphere comprising a first atmosphere and a second atmosphere, wherein the first atmosphere is selected from at least one of air, nitrogen, carbon monoxide and carbon dioxide, and the second atmosphere is selected from steam, wherein the volume ratio of the first atmosphere to the second atmosphere is (1-10):1, for example (5-9):1 or (1-2):1, the temperature for the heat treatment ranges from 300 to 1200 °C, and the time for the heat treatment ranges from 1 to 24 h, preferably 1 to 7 h.

According to the present invention, the crystallization condition can be selected within a wide range. In one embodiment of the present invention, a condition of the crystallization includes at least one of the following: a temperature of 150-300 °C, for example 150-250 °C; a duration of 2-24 hours, preferably 2-20 hours; a pressure of 0.2-10 MPa, preferably 2-5 MPa; water as the solvent; a pH of a solution for the crystallization of 8-14, preferably 9-11.

In one embodiment of the present invention, a condition of the irradiation includes: a ray source selected from y rays; and/or an irradiation dose of 1-20 kGy, preferably 8-18 kGy.

In one embodiment of the present invention, the amount of the inorganic carbon is 1-30 parts by mass, preferably 3-20 parts by mass, based on 100 parts by mass of the carrier (e.g., alumina) precursor.

According to the present invention, when the modified carrier is alumina, the alumina precursor can be selected within a broad range. In one embodiment of the present invention, the alumina precursor is selected from at least one of pseudoboehmite, α-alumina, θ-alumina, γ-alumina and amorphous alumina, for example at least one of pseudoboehmite, α-alumina and γ-alumina.

According to the present invention, the molding pore-expanding agent can be selected within a broad range. In one embodiment of the present invention, the molding pore-expanding agent is selected from at least one of polyethylene glycol cellulose, methyl cellulose, carboxymethyl cellulose, ethyl cellulose, hydroxyethyl cellulose and starch, and selected from at least one of polyvinyl alcohol, polyethylene glycol, polyacrylamide, polypropylene glycol and sesbania powder.

According to the present invention, the ratio of the mass of the molding pore-expanding agent to the total mass of the dry material can be selected within a broad range. In one embodiment of the present invention, the ratio of the mass of the molding pore-expanding agent to the total mass of the dry material is (0.5-25):100, preferably (5-13):100.

In one embodiment of the present invention, the liquid is an acidic solution. Preferably, the acidic solution is selected from at least one of aqueous solutions of acetic acid, ethanedioic acid, diacetic acid, maleic acid, citric acid, oxalic acid, formic acid, tartaric acid, nitric acid, sulfuric acid, hydrochloric acid, phosphoric acid and hypochlorous acid, and/or the acidic solution has a concentration of 0.01-5 mol/L, preferably 0.2-4 mol/L.

A fifth aspect of the present invention is to provide a use of the catalyst according to the first aspect or the catalyst prepared by the preparation method according to the second aspect in a catalytic hydrogenation field, preferably in a catalytic hydrogenation of acetylene to prepare ethylene.

A sixth aspect of the present invention is to provide a use of the modified carrier according to the third aspect or the modified carrier prepared by the preparation method according to the fourth aspect in the preparation of an eggshell catalyst.

Compared with the prior art, the present invention has the advantages of:

(1) the catalyst of the present invention, in which 95 wt% to 99 wt% of the active metal element relative to the total weight of the active metal element are distributed in the shell layer of the carrier with a thickness of 60 μm or less. The catalyst has a thinner shell layer than the conventional eggshell-type catalysts and is more beneficial to the rapid surface reactions and the diffusion-controlled catalytic reactions;
(2) the catalyst of the present invention, in which the active metal element has a dispersity of 50% to 80%. The catalyst has a higher dispersity than the conventional eggshell-type catalysts, and a higher atom utilization rate of the active metal, which can significantly reduce the cost of the catalyst;
(3) the present invention can prepare the catalyst using a specific alumina carrier with a relatively high content and density of the oxygen-containing group, which has relatively low requirements for the impregnation condition. Meanwhile the rich type and the high density of the oxygen-containing group on the alumina carrier surface can allow the active metal to be distributed in an extremely thin shell layer region on the carrier surface while weakening their growth, agglomeration or aggregation trend, and further exhibiting a highly dispersed state, whereby the regulation of the catalyst is more precise and accurate.
(4) The catalyst of the present invention exhibits excellent performances in selective catalytic hydrogenation reactions.

## DESCRIPTION OF THE DRAWINGS

Figure 1 shows the SEM result of the active metal palladium of catalyst X1 in Example 1.
Figure 2 shows the SEM result of the active metal palladium of catalyst Y1 in Comparative Example 1.

## MODE OF CARRYING OUT THE INVENTION

The present invention will be further described in detail through specific examples. It is necessary to indicate here that the following examples are only used to further describe the present invention and cannot be understood as a limitation to the protection scope of the present invention. Some non-essential improvements and adjustments made to the present invention by those skilled in the art based on the contents of the present invention still fall within the protection scope of the present invention.

I. Preparation examples

Example 1

200 g of pseudoboehmite powder (having a pore volume of 0.6 ml/g and a specific surface area of 208.5 $m^2/g$), 5 g of methyl cellulose, 7 g of polypropylene glycol and 8 g of graphene were mixed evenly. 20 ml of a mixed aqueous solution of 1 mol/L nitric acid and 0.5 mol/L citric acid was added to the mixed powder. Then the mixture was fully kneaded, extruded, molded and pelletized to obtain spherical particles with a particle size of 3 mm. The particles were dried at 120 °C for 4 hours, followed by a heat treatment at 500 °C for 2 hours in a mixed atmosphere of carbon monoxide and steam (having a volume ratio of 5:1), and then crystallized in an aqueous solution with a pH of 9.8 at 200 °C and 2 MPa for 2 hours to obtain a carrier A1. Next, 50 g of A1 was impregnated with 60 ml of a palladium chloride solution containing 0.025 g of palladium, and dried at 120 °C for 4 hours after letting stand for 2 hours, and calcined in a flowing hydrogen atmosphere at 400 °C for 12 hours to obtain a catalyst X1 with a palladium loading amount of 0.05 wt%.

Example 2

100 g of $\alpha$-alumina (having a pore volume of 1.7 ml/g and a specific surface area of 79.5 $m^2/g$) and 50 g of amorphous alumina (having a pore volume of 1.1 ml/g and a specific surface area of 86.2 $m^2/g$), 5 g of methyl cellulose, 7 g of polyethylene glycol, 8 g of coconut shell charcoal, 5.3 g of indium nitrate and 4 g of zinc nitrate were mixed evenly. 40 ml of a mixed aqueous solution of 2 mol/L oxalic acid and 1 mol/L acetic acid was added to the mixed powder. Then the mixture was fully kneaded, extruded, molded and pelletized to obtain cylindrical particles with a particle size of 4 mm. The particles were dried at 150 °C for 2 hours, followed by a $\gamma$-ray irradiation for 10 kGy, and then crystallized in an aqueous solution with a pH of 11 at 240 °C and 2 MPa for 2 hours to obtain a carrier A2. Next, 50 g of A2 was impregnated with 50 ml of a palladium chloride solution containing 0.025 g of palladium, and dried at 150 °C for 6 hours after letting stand for 1 hour, and calcined in a flowing mixed atmosphere of nitrogen and argon at 550 °C for 6 hours to obtain a catalyst X2 with a palladium loading amount of 0.05 wt%.

Example 3

100 g of $\alpha$-alumina (having a pore volume of 0.9 ml/g and a specific surface area of 48.2 $m^2/g$) and 50 g of $\theta$-alumina (having a pore volume of 1.8 ml/g and a specific surface area of 106.3 $m^2/g$), 5 g of methyl cellulose, 5 g of starch, 5 g of polypropylene glycol, 5 g of sesbania powder, 5 g of bamboo charcoal and 5 g of coconut shell charcoal were mixed evenly. 45 ml of a 1.5 mol/L sulfuric acid solution was added to the mixed powder. Then the mixture was fully kneaded, extruded, molded and pelletized to obtain spherical particles with a particle size of 3 mm. The particles were dried at 120 °C for 4 hours, heat treated at 850 °C for 6 hours in a mixed atmosphere of carbon dioxide and steam (having a volume ratio of 9:1), followed by a $\gamma$-ray irradiation for 8 kGy to obtain a carrier A3. Next, 50 g of A3 was loaded with 35 ml of a palladium nitrate solution containing 0.05 g of palladium, and dried at 120 °C for 2 hours after letting stand for 5 hours, and calcined in a flowing mixed atmosphere of air at 450 °C for 2 hours to obtain a catalyst X3 with a palladium loading amount of 0.1 wt%.

Example 4

100 g of $\alpha$-alumina (having a pore volume of 0.9 ml/g and a specific surface area of 48.2 $m^2/g$) and 50 g of $\gamma$-alumina (having a pore volume of 1.5 ml/g and a specific surface area of 92.7 $m^2/g$), 5 g of carboxymethyl cellulose, 5 g of hydroxyethyl cellulose, 5 g of sesbania powder, 5 g of diamond and 5 g of graphdiyne were mixed evenly. 30 mL of a mixed aqueous solution of 0.5 mol/L hydrochloric acid and 0.2 mol/L hypochlorous acid was added to the mixed powder. Then the mixture was fully kneaded, extruded, molded and pelletized to obtain spherical particles with a particle size of 3 mm. The particles were dried at 110 °C for 4 hours, then crystallized in an aqueous solution with a pH of 10 at 245 °C and 5 MPa for 20 hours, dried at 130 °C for 4 hours, followed by a $\gamma$-ray irradiation for 8 kGy and then a heat treatment at 350 °C for 6 hours in a mixed atmosphere of carbon monoxide, carbon dioxide and steam (having a volume ratio of 6:2:1) to obtain a carrier A4. Next, 50 g of A4 was loaded with 25 ml of a palladium nitrate solution containing 0.15 g of palladium and irradiated with a $\gamma$-ray for 5 kGy to obtain a catalyst X4 with a palladium loading amount of 0.3 wt%.

Example 5

80 g of amorphous alumina (having a pore volume of 1.1 ml/g and a specific surface area of 86.2 $m^2/g$) and 40 g of pseudoboehmite (having a pore volume of 0.6 ml/g and a specific surface area of 208.5 $m^2/g$), 10 g of starch, 10 g of sesbania powder, 6 g of bamboo charcoal, 6 g of wood charcoal and 6 g of coconut shell charcoal were mixed evenly. A mixed aqueous solution of 1.5 mol/L formic acid, 1.5 mol/L maleic acid and 0.5 mol/L diacetic acid was added to the mixed powder. Then the mixture was fully kneaded, extruded, molded and pelletized to obtain spherical particles with a particle size of 3 mm. The particles were dried at 110 °C for 4 hours, irradiated with a $\gamma$-ray for 18 kGy and then heat treated at 950 °C

for 1.5 hours in a mixed atmosphere of nitrogen, carbon dioxide and steam (having a volume ratio of 4:5:1) to obtain a carrier A5. Next, 50 g of A5 was loaded with 30 ml of a mixed aqueous solution of palladium nitrate containing 0.04 g of palladium and silver nitrate containing 0.04 g of silver, let stand for 4 hours, and calcined in a flowing atmosphere of air at 650 °C for 8 hours to obtain a catalyst X5 with a palladium loading amount of 0.08 wt%.

Example 6

80 g of amorphous alumina (having a pore volume of 1.1 ml/g and a specific surface area of 86.2 $m^2$/g) and 40 g of pseudoboehmite (having a pore volume of 0.6 ml/g and a specific surface area of 208.5 $m^2$/g), 5 g of starch, 5 g of carboxymethyl cellulose, 5 g of sesbania powder, 6 g of polyvinyl alcohol, 10 g of wood charcoal and 12 g of graphene were mixed evenly. 50 mL of a 3.5 mol/L phosphoric acid solution was added to the mixed powder. Then the mixture was fully kneaded, extruded, molded and pelletized to obtain spherical particles with a particle size of 3 mm. The particles were dried at 110 °C for 4 hours, crystallized in an aqueous solution with a pH of 10 at 280 °C and 0.8 MPa for 2 hours, and then heat treated at 1050 °C for 6 hours in a mixed atmosphere of air, carbon monoxide and steam (having a volume ratio of 0.25:8:1) to obtain a carrier A6. Next, 50 g of A6 was loaded with 30 ml of a mixed aqueous solution of palladium oxalate containing 0.03 g of palladium, let stand for 4 hours, and calcined in a flowing nitrogen atmosphere at 400 °C for 8 hours to obtain a catalyst X6 with a palladium loading amount of 0.04 wt%.

Comparative Example 1

200 g of pseudoboehmite powder (having a pore volume of 0.6 ml/g and a specific surface area of 208 $m^2$/g), 5 g of methyl cellulose and 7 g of polypropylene glycol were mixed evenly. 20 ml of a mixed aqueous solution of 1 mol/L nitric acid and 0.5 mol/L citric acid was added to the mixed powder. Then the mixture was fully kneaded, extruded, molded and pelletized to obtain spherical particles with a particle size of 3 mm. The particles were dried at 120 °C for 4 hours, then heat treated at 500 °C for 2 hours in a mixed atmosphere of carbon monoxide and steam (having a volume ratio of 5:1), and further crystallized in an aqueous solution with a pH of 9.8 at 200 °C and 2 MPa for 2 hours to obtain a carrier B1. Next, 50 g of B1 was impregnated with 60 ml of a palladium chloride solution containing 0.025 g of palladium, and dried at 120 °C for 4 hours after letting stand for 2 hours, and calcined in a flowing hydrogen atmosphere at 400 °C for 12 hours to obtain a catalyst Y1 with a palladium loading amount of 0.05 wt%.

Comparative Example 2

200 g of pseudoboehmite powder (having a pore volume of 0.6 ml/g and a specific surface area of 208 $m^2$/g), 5 g of methyl cellulose, 7 g of polypropylene glycol and 8 g of graphene were mixed evenly. 20 ml of a mixed aqueous solution of 1 mol/L nitric acid and 0.5 mol/L citric acid was added to the mixed powder. Then the mixture was fully kneaded, extruded, molded and pelletized to obtain spherical particles with a particle size of 3 mm. The particles were dried at 120 °C for 4 hours, followed by calcination at 500 °C for 2 hours in an air atmosphere to obtain a carrier B2. Next, 50 g of B2 was impregnated with 60 ml of a palladium chloride solution containing 0.025 g of palladium, and dried at 120 °C for 4 hours after letting stand for 2 hours, and calcined in a flowing hydrogen atmosphere at 400 °C for 12 hours to obtain a catalyst Y2 with a palladium loading amount of 0.05 wt%.

Comparative Example 3

100 g of $\alpha$-alumina (having a pore volume of 1.7 ml/g and a specific surface area of 79.5 $m^2$/g) and 50 g of amorphous alumina (having a pore volume of 1.1 ml/g and a specific surface area of 86.2 $m^2$/g), 5 g of methyl cellulose, 7 g of polyethylene glycol, 8 g of coconut shell charcoal, 5.3 g of indium nitrate and 4 g of zinc nitrate were mixed evenly. 40 ml of a mixed aqueous solution of 2 mol/L oxalic acid and 1 mol/L acetic acid was added to the mixed powder. Then the mixture was fully kneaded, extruded, molded and pelletized to obtain cylindrical particles with a particle size of 4 mm. The particles were dried at 150 °C for 2 hours, followed by a $\gamma$-ray irradiation for 10 kGy to obtain a carrier B3. Next, 50 g of B3 was impregnated with 50 ml of a palladium chloride solution containing 0.025 g of palladium, and dried at 150 °C for 6 hours after letting stand for 1 hour, and calcined in a flowing mixed atmosphere of nitrogen and argon at 550 °C for 6 hours to obtain a catalyst Y3 with a palladium loading amount of 0.05 wt%.

Comparative Example 4

100 g of $\alpha$-alumina (having a pore volume of 0.9 ml/g and a specific surface area of 48.2 $m^2$/g) and 50 g of $\gamma$-alumina (having a pore volume of 1.5 ml/g and a specific surface area of 92.7 $m^2$/g), 5 g of carboxymethyl cellulose, 5 g of hydroxyethyl cellulose, 5 g of sesbania powder, 5 g of diamond and 5 g of graphdiyne were mixed evenly. 30 mL of a mixed

aqueous solution of 0.5 mol/L hydrochloric acid and 0.2 mol/L hypochlorous acid was added to the mixed powder. Then the mixture was fully kneaded, extruded, molded and pelletized to obtain spherical particles with a particle size of 3 mm. The particles were dried at 110 °C for 4 hours, then crystallized in an aqueous solution with a pH of 10 at 245 °C and 5 MPa for 20 hours, and dried at 130 °C for 4 hours to obtain a carrier B4. Next, 50 g of B4 was loaded with 25 ml of a palladium nitrate solution containing 0.15 g of palladium, followed by a γ-ray irradiation for 5 kGy to obtain a catalyst Y4 with a palladium loading amount of 0.3 wt%.

Comparative Example 5

80 g of amorphous alumina (having a pore volume of 1.1 mL/g and a specific surface area of 86.2 $m^2$/g) and 40 g of pseudoboehmite (having a pore volume of 0.6 mL/g and a specific surface area of 208.5 $m^2$/g), 10 g of starch, 10 g of sesbania powder, 6 g of bamboo charcoal, 6 g of wood charcoal and 6 g of coconut shell charcoal were mixed evenly. A mixed aqueous solution of 1.5 mol/L formic acid, 1.5 mol/L maleic acid and 0.5 mol/L diacetic acid was added to the mixed powder. Then the mixture was fully kneaded, extruded, molded and pelletized to obtain spherical particles with a particle size of 3 mm. The particles were dried at 110 °C for 4 hours, followed by a heat treatment at 950 °C for 1.5 hours in a mixed atmosphere of nitrogen, carbon dioxide and steam (having a volume ratio of 4:5:1) to obtain a carrier B5. Next, 50 g of B5 was loaded with 30 ml of a mixed aqueous solution of palladium nitrate containing 0.04 g of palladium and silver nitrate containing 0.04 g of silver, let stand for 4 hours, and calcined at 650 °C for 8 hours in a flowing atmosphere of air to obtain a catalyst Y5 with a palladium loading amount of 0.08 wt%.

Comparative Example 6

80 g of trilobe extrudate alumina with a pore volume of 1.2 mL/g, a specific surface area of 180 $m^2$/g and a length of 4 mm was used as a carrier B6. About 7 mL of sodium hydroxide was added to a palladium nitrate solution (obtained by diluting 1 g of a palladium nitrate solution containing 8.0 wt% palladium with 45 mL of softened water) at 25 °C under stirring, obtaining a pH of 2.4 to prepare a colloidal suspension of palladium oxide. Then the suspension was diluted with softened water to a volume corresponding to the pore volume of the carrier B6. Next the solution was impregnated onto the selected alumina carrier. The impregnated carrier was aged for 20 hours in a closed humid medium in air. The resultant was dried at 120 °C for 2 hours in air and calcined at 450 °C for 2 hours in a flowing atmosphere of air to obtain a catalyst Y6 with a palladium loading amount of 0.1 wt%.

Comparative Example 7

80 g of trilobe extrudate alumina with a pore volume of 1.2 mL/g, a specific surface area of 180 $m^2$/g and a length of 4 mm was used as a carrier B7. About 7 mL of sodium hydroxide was added to a palladium nitrate solution (obtained by diluting 0.1 g of a palladium nitrate solution containing 8.0 wt% palladium with 45 mL of softened water) at 25 °C under stirring, obtaining a pH of 2.4 to prepare a colloidal suspension of palladium oxide. Then the suspension was diluted with softened water to a volume corresponding to the pore volume of the carrier B7. Next the solution was impregnated onto the selected alumina carrier. The impregnated carrier was aged for 20 hours in a closed humid medium in air. The resultant was dried at 120 °C for 2 hours in air and calcined at 450 °C for 2 hours in a flowing atmosphere of air to obtain a catalyst Y7 with a palladium loading amount of 0.01 wt%.

II. Detection examples

Determination of the physical property of the specific surface area of the carrier: the measurement is carried out by a BET method of nitrogen physical adsorption.

Determination of density of the oxygen-containing group of the carrier: the property and the amount of the oxygen-containing group of the carrier were determined by using the Boehm's chemical method (details can be seen in H. P. Boehm, "Some Aspects of the Surface Chemistry of Carbon Blacks and Other Carbons", Carbon, 1994). Three carrier samples of 1 g were weighed and respectively immersed in 50 mL of 0.05 mol/L $NaHCO_3$, $Na_2CO_3$ and NaOH solutions for 24 hours. Then, 10 mL of the immersed solution was titrated with 0.06 mol/L hydrochloric acid. Each sample was titrated three times, and the arithmetic mean value was taken. The amount of various types of oxygen-containing groups on the carrier surface was calculated based on the alkali consumption amount, and the density of the oxygen-containing group was obtained by combining with the data of the specific surface area of the carrier.

Determination of the active metal distribution on the catalyst surface: the catalyst sample particle was cut in half, and the active element distribution in the cross-section of the particle was analyzed by using a JSM-7900F scanning electron microscope equipped with an Energy Disperse Spectroscopy EDX elemental analysis attachment. The shell layer thickness L corresponding to the active metal content of over 95% in the shell layer range where the active metal

content is below 0.01% from the exterior to the interior of the cross-section was calculated (the average value was taken from the measurements at three different locations on each cross-section).

Determination of the palladium content in the catalyst: a conventional ICP-MS method was used to detect the content of palladium metal in the catalyst.

Determination of the dispersity of the active metal in the catalyst: by using a Chemisorb 2920 chemisorption instrument, 0.1 g of the catalyst sample was weighed, reduced with hydrogen at 160 °C for 30 min, purged with nitrogen for 10 min, then cooled to 40 °C, and subjected to a CO pulse adsorption until saturation. The dispersity of the active metal was calculated through the measurement result of the adsorbed amount and the palladium content.

Determination of the particle size distribution of the active metal in the catalyst: the catalyst sample particle was cut at a thickness of about 0.5 mm on the surface, ground and then reduced with hydrogen at 160 °C for 1 hour. Then the powder sample was added in a solution of water and ethanol with a volume ratio of 4:1, and dispersed by ultrasonication. Next, 4 to 6 drops of the dispersed solution were taken with a dropper and dripped onto a copper net, dried under an infrared lamp for 15 minutes and then directly transferred to an electron microscope. Four images were taken for each sample, and the particle sizes of at least 200 particles in total were calculated.

Catalytic performance test: 10 mL of the catalyst was loaded into a tubular reactor, and reduced at 160 °C for 3 hours by hydrogen introduced after nitrogen displacement; after naturally cooling to the room temperature, a purgation was carried out with nitrogen for displacement. A performance test (Test 1) was conducted in a C2 reaction material (Material 1) at a space velocity of 20,000 $h^{-1}$ and a pressure of 2 MPa; the above steps and the conditions were repeated, and a performance test (Test 2) was conducted in a C2 reaction material (Material 2):

Components of Material 1: 1000 ppm of acetylene, 800 ppm of carbon monoxide, 13% of hydrogen, 12% of ethane, 1% of methane, 20% of propylene, 0.3% of propadiene, 0.2% of propyne, 0.9% of propane, and the balance of ethylene.

Components of Material 2: 1000 ppm of acetylene, 900 ppm of carbon monoxide, 26% of hydrogen, 14% of ethane, 3% of methane, and the balance of ethylene.

The lowest temperature and the ethylene selectivity corresponding to the acetylene concentration of 0 ppm in the outlet gas were investigated.

The ethylene selectivity is calculated by the following formula:

ethylene selectivity = [(ethylene content at outlet - ethylene content at inlet)/(acetylene content at inlet)] × 100%

Table 1 Measurement results of the oxygen-containing group in the alumina carrier in the examples and the comparative examples

| Number | Specific surface area ($m^2/g$) | Hydroxyl group (mmol/g) | Carboxyl group (mmol/g) | Lactone group (mmol/g) | Density of the oxygen-containing group ($mmol/m^2$) |
|---|---|---|---|---|---|
| A1 | 77.1 | 7.45 | 13.21 | 6.42 | 0.351 |
| A2 | 63.5 | 11.52 | 7.28 | 8.79 | 0.434 |
| A3 | 84.3 | 9.75 | 8.21 | 5.8 | 0.282 |
| A4 | 92.1 | 6.52 | 6.57 | 3.51 | 0.180 |
| A5 | 120.6 | 24.23 | 15.75 | 10.55 | 0.419 |
| A6 | 35.2 | 13.20 | 8.31 | 8.12 | 0.842 |
| B1 | 73.5 | 1.77 | 1.24 | 0.64 | 0.050 |
| B2 | 69.4 | 1.45 | 1.01 | 0.09 | 0.037 |
| B3 | 66.9 | 2.61 | 1.74 | 0.55 | 0.073 |
| B4 | 98.6 | 3.77 | 2.42 | 0.58 | 0.069 |
| B5 | 119.5 | 4.51 | 3.25 | 1.44 | 0.077 |
| B6 | 175.7 | 1.58 | 0.79 | 0.11 | 0.014 |
| B7 | 175.7 | 1.58 | 0.79 | 0.11 | 0.014 |

Table 2 Test results of the physical and chemical property characterization of the catalysts in the examples and the comparative examples

| Number | Palladium content (wt%) | Dispersity (%) | Thickness L (μm) | Average particle size of palladium particle (nm) |
|---|---|---|---|---|
| X1 | 0.05 | 60.6 | 45.2 | 3.1 |
| X2 | 0.05 | 69.3 | 49.6 | 2.9 |
| X3 | 0.1 | 70.1 | 40.2 | 2.7 |
| X4 | 0.3 | 55.6 | 38.5 | 3.6 |
| X5 | 0.08 | 72.4 | 51.3 | 2.7 |
| X6 | 0.04 | 76.7 | 56.8 | 2.4 |
| Y1 | 0.05 | 38.7 | 104.6 | 5.6 |
| Y2 | 0.05 | 32.6 | 74.3 | 5.9 |
| Y3 | 0.05 | 40.1 | 65.2 | 4.7 |
| Y4 | 0.3 | 24.3 | 119.4 | 7.8 |
| Y5 | 0.08 | 29.8 | 64.1 | 8.2 |
| Y6 | 0.1 | 3.2 | 45.2 | 15.3 |
| Y7 | 0.01 | 8.7 | 34.7 | 14.5 |

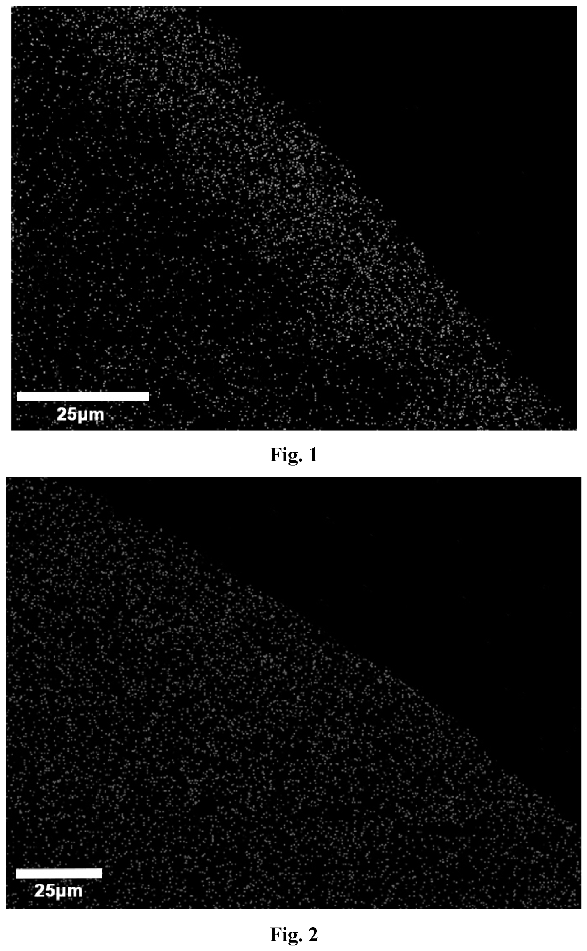

It can be seen from Table 1 that the carrier modification method of the present invention can significantly increase the content and the density of the oxygen-containing group on the surface of the alumina carrier. Moreover, it can be seen from Table 2 that compared with the catalysts in the comparative examples, the active metals of the catalysts prepared by the method of the present invention can simultaneously satisfy the effects that a dispersity of greater than 55% and an average particle size of smaller than 4 nm are achieved in an extremely thin (less than 60 μm) shell layer. Please also refer to the comparison of Figure 1 and Figure 2.

Table 3 Evaluation results of the catalytic performances of the catalysts in the examples and the comparative examples

| Number | Test 1 | | Test 2 | |
|---|---|---|---|---|
| | Temperature (°C) | Selectivity (%) | Temperature (°C) | Selectivity (%) |
| X1 | 58.7 | -97.6 | 56.3 | -95.2 |
| X2 | 56.6 | -88.5 | 53.8 | -84.3 |
| X3 | 52.8 | -76.2 | 51.2 | -80.1 |
| X4 | 46.2 | -83.6 | 43.7 | -82.5 |
| X5 | 57.5 | -71.6 | 55.3 | -68.5 |
| X6 | 62.4 | -74.3 | 60.5 | -73.9 |
| Y1 | 69.8 | -129.4 | 66.1 | -130.3 |
| Y2 | 70.6 | -116.5 | 69.4 | -114.2 |
| Y3 | 74.5 | -120.4 | 72.1 | -118.5 |
| Y4 | 57.6 | -142.8 | 55.1 | -140.7 |
| Y5 | 70.2 | -118.4 | 67.5 | -114.1 |
| Y6 | 72.3 | -112.7 | 68.7 | -109.7 |
| Y7 | 90.7 | -122.3 | 88.1 | -124.6 |

Results in Table 3 demonstrate that under the same condition of achieving an acetylene outlet concentration of 0 ppm, i.e., when the acetylene conversion is 100%, the temperatures required by the catalysts in the examples are lower

than those of the corresponding catalysts in the comparative examples, which shows that the catalysts in the examples have better activities. Moreover, when the acetylene conversions are all 100%, the selectivities of the catalysts in the examples are also better than those of the corresponding catalysts in the comparative examples. Therefore, the catalysts in the examples have better performances than the catalysts in the comparative examples, and achieve unexpected technical effects.

In two typical C2 selective hydrogenation reactions (Table 3), the activities and the selectivities of the catalysts in the examples are significantly better than those of the catalysts in the comparative examples. This may be attributed to the fact that the C2 selective hydrogenation reaction is a rapid surface reaction and a diffusion-controlled catalytic reaction. The more the active metal of the catalyst is enriched in the outer layer of the catalyst and the higher the dispersity (atomic utilization rate) is, the better the activity of the catalyst is. Moreover, the desorption of ethylene is easier, with a shorter diffusion path from the catalyst surface to the gas phase and a lower probability of over-hydrogenation, and thus the selectivity of the catalyst is also higher.

It should be noted that the above examples are only used to explain the present invention and do not form any limitation to the present invention. The present invention is described by referring to the typical examples, but it should be understood that the words used therein are descriptive and explanatory vocabulary, rather than restrictive vocabulary. The present invention may be amended within the scopes of the claims of the present invention according to the stipulations, and may be revised without deviating from the scope and the spirit of the present invention. Although the present invention described herein relates to specific methods, materials, and examples, it does not mean that the present invention is limited to the specific examples disclosed herein. On the contrary, the present invention can be extended to all other methods and uses with the same function.

All the publications, the patent applications, the patents and other reference literatures mentioned in the present description are incorporated herein by reference. Unless otherwise defined, all the technical and scientific terms used in the present description have the meanings conventionally understood by those skilled in the art. In case of conflict, the definitions in the present description shall prevail.

When materials, substances, methods, steps, devices or parts etc. are derived in the present description using the prefix "known to those skilled in the art", "prior art", or similar terms, the objects derived from the prefix cover those conventionally used in the art at the time of filing of the present application, and also include those that are not commonly used currently but will become applicable for similar purposes as generally recognized in the art.

None of the endpoints and the values of the ranges disclosed in the documents of the present application is limited to the precise ranges or values, and these ranges or values should be understood to include the values close to these ranges or values. Value ranges between the endpoint values of each range, between the endpoint values and the individual point values of each range and between the individual point values can be combined with each other to obtain one or more new value ranges, which should be considered as specifically disclosed in the present application. In the following text, various technical solutions can be combined with each other in principle to obtain new technical solutions, which should also be considered as specifically disclosed in the present application.

In the context of the present description, besides the explicitly stated contents, any unmentioned matter is directly applicable to those known in the art without requiring any change.

Moreover, any embodiment described in the present application can be arbitrarily combined with one or more other embodiments described in the present application. The technical solution or the technical idea formed therefrom belongs to a part of the original disclosure of the present description, but should not be considered as new contents that have not been disclosed or expected in the present application, unless those skilled in the art believe that the combination is obviously unreasonable.

## Claims

**1.** An eggshell-type catalyst comprising a carrier and an active metal element, wherein 95 wt% to 99 wt% of the active metal element relative to the total weight of the active metal element are distributed in the shell of the carrier with a thickness of 60 μm or less, calculated from an outer surface of the carrier as a starting point toward a center, and exhibit an eggshell-type distribution, and
wherein the active metal element has a dispersity of 50% to 80%.

**2.** The eggshell-type catalyst according to claim 1, wherein

the active metal element comprises a main metal element and optionally an auxiliary metal element; and/or
95 wt% to 99 wt% of the active metal element relative to the total weight of the active metal element are distributed in the shell of the carrier with a thickness of 50 μm or less, calculated from the outer surface of the carrier as a starting point toward the center; and/or

the active metal element has a dispersity of 60% to 80%, preferably greater than 70%, more preferably 71% to 80%; and/or
the active metal has an average particle size of less than 4 nm, preferably of 2-3.7 nm; and/or
the catalyst has a specific surface area of 20-150 $m^2$/g.

3. The eggshell-type catalyst according to claim 2, wherein

the ratio of the weight of the main metal element to the weight of the carrier is (0.01-0.5):100, preferably (0.05-0.3):100, based on the weight of the main metal element; and/or
the main metal element is selected from at least one of metal elements of Group VIIIB, preferably palladium; and/or
the ratio of the total weight of the auxiliary metal element to the weight of the carrier is (0-5):100, based on the weight of the auxiliary metal element; and/or
the auxiliary metal element is selected from at least one of metal elements of Group IB, Group IIB, Group IIIA, Group IIIA and Lanthanide, preferably at least one of silver, indium, gallium, cerium and zinc.

4. A method for preparing the catalyst according to any one of claims 1 to 3, comprising:
impregnating a modified carrier with a solution containing a source of an active metal element followed by activation to obtain the catalyst, wherein a surface of the modified carrier has an oxygen-containing group with a density of 0.1-1.2 mmol/$m^2$, preferably 0.1-0.9 mmol/$m^2$, more preferably 0.15-0.9 mmol/$m^2$, and still more preferably 0.18-0.85 mmol/$m^2$.

5. The preparation method according to claim 4, wherein

the oxygen-containing group is at least one of hydroxyl, carboxyl and a lactone group, and more preferably, the oxygen-containing group is hydroxyl, carboxyl and a lactone group,
more preferably, a content of hydroxyl, carboxyl and the lactone group is:

the content of the hydroxyl is 4-40 mmol/g, preferably 6-30 mmol/g; and/or
the content of the carboxyl is 2.5-30 mmol/g, preferably 6-20 mmol/g; and/or
the content of the lactone group is 0.05-20 mmol/g, preferably 1.5-20 mmol/g.

6. The preparation method according to claim 4 or 5, wherein

the source of the active metal element comprises a source of the main metal element and optionally a source of an auxiliary metal element,
wherein preferably the source of the main metal element is selected from at least one of soluble compounds containing the main metal element (e.g., a metal element of Group VIIIB), preferably at least one of water-soluble palladium compounds, more preferably at least one of palladium nitrate, palladium chloride, sodium chloropalladate, palladium oxalate, and palladium citrate, and the ratio of the content of the source of the main metal element (e.g., a palladium compound), calculated as a metal element, to the weight of the carrier is (0.01-0.5):100, preferably (0.02-0.45):100; and/or
wherein preferably the source of the auxiliary metal element is selected from at least one of soluble compounds comprising an auxiliary metal element (e.g. metal elements of Group IB, Group IIB, Group IIIA, Group IIIA and Lanthanide), preferably water-soluble salt compounds of the auxiliary metal element, for example, a compound of the auxiliary metal is at least one of soluble salt compounds of silver, indium, gallium, cerium and zinc, and the ratio of the content of the source of the auxiliary metal element (the compound of the auxiliary metal), calculated as a metal element, to the weight of the carrier may be (0-5):100, preferably (0.02-4):100.

7. The preparation method according to any one of claims 4 to 6, wherein

the modified carrier is at least one of alumina, magnesia, calcium oxide, hydrotalcite, silica, titania, ceria, preferably alumina, and/or
the modified carrier has a specific surface area of 20-150 $m^2$/g, preferably 30-130 $m^2$/g, and/or
an impregnation solution used is an aqueous solution of a palladium compound or a compound of the auxiliary metal, or a mixture of aqueous solutions of the above two compounds; and/or
the impregnation is carried out once or multiple times, wherein the activation is optionally carried out before the impregnation, after the impregnation, or at intervals between multiple times of the impregnation; preferably, when

the activation comprises multiple stages, some stages of the activation are optionally conducted before the impregnation, after the impregnation, or at intervals between multiple times of the impregnation; and/or
the activation step can be carried out in any suitable sequence; and/or
the activation method comprises a calcination stage, or an irradiation stage, or both of the calcination stage and the irradiation stage carried out in any sequence, wherein preferably, a condition of the calcination comprises:

a temperature of 150-750 °C, and/or
a duration of 2-20 h, e.g., 4-20 h, and/or
an atmosphere of at least one of air, nitrogen, hydrogen, argon; and/or
preferably, an irradiation condition in the irradiation stage comprises: a ray source selected from a γ ray, and/or an irradiation dose of 1-20 kGy, preferably 4-18 kGy.

**8.** A modified carrier for an eggshell-type catalyst, wherein a surface of the modified carrier has an oxygen-containing group with a density of 0.1-1.2 mmol/$m^2$, preferably 0.1-0.9 mmol/$m^2$, more preferably 0.15-0.9 mmol/$m^2$, and still more preferably 0.18-0.85 mmol/$m^2$.

**9.** The modified carrier according to claim 8, wherein

the oxygen-containing group is at least one of hydroxyl, carboxyl and a lactone group, and preferably, the oxygen-containing group is hydroxyl, carboxyl and a lactone group,
more preferably, a content of hydroxyl, carboxyl and the lactone group is:

the content of the hydroxyl is 4-40 mmol/g, preferably 6-30 mmol/g; and/or
the content of the carboxyl is 2.5-30 mmol/g, preferably 6-20 mmol/g; and/or
the content of the lactone group is 0.05-20 mmol/g, preferably 1.5-20 mmol/g.

**10.** The modified carrier according to claim 8 or 9, wherein

the modified carrier is at least one of alumina, alumina, magnesia, calcium oxide, hydrotalcite, silica, titania, ceria, preferably alumina, and/or
the modified carrier has a specific surface area of 20-150 $m^2$/g, preferably 30-130 $m^2$/g.

**11.** A method for preparing the modified carrier according to any one of claims 8 to 10, comprising mixing a dry material including a carrier precursor, a molding pore-expanding agent and an inorganic carbon with a liquid, followed by kneading, granulation and a surface modification to obtain the modified carrier, wherein the surface modification involves at least two methods of heat treatment, crystallization and irradiation.

**12.** The preparation method according to claim 11, wherein

the inorganic carbon is selected from at least one of wood charcoal, bamboo charcoal, coconut shell charcoal, graphene, graphyne and diamond; and/or
the amount of the inorganic carbon is 1-30 parts by mass, preferably 3-20 parts by mass, based on 100 parts by mass of the carrier precursor; and/or
the surface modification involves crystallization and heat treatment, irradiation and heat treatment, or irradiation and crystallization, preferably crystallization and heat treatment, or irradiation and heat treatment; and/or
a condition of the heat treatment includes a treatment with a heat treatment atmosphere comprising a first atmosphere selected from at least one of air, nitrogen, carbon monoxide and carbon dioxide and a second atmosphere selected from steam, wherein a volume ratio of the first atmosphere to the second atmosphere is (1-10):1, for example (5-9):1 or (1-2):1, the temperature for the heat treatment ranges from 300 to 1200 °C, and the time for the heat treatment ranges from 1 to 24 h, preferably 1 to 7 h; and/or
a condition of the crystallization includes at least one of the following: a temperature of 150-300 °C, for example 150-250 °C; a duration of 2-24 hours, preferably 2-20 hours; a pressure of 0.2-10 MPa, preferably 2-5 MPa; a solvent of water; a pH of a solution for the crystallization of 8-14, preferably 9-11; and/or
a condition of the irradiation includes: a ray source selected from a γ ray; and/or an irradiation dose of 1-20 kGy, preferably 8-18 kGy.

**13.** The preparation method according to claim 11 or 12, wherein

when the modified carrier is alumina, the alumina precursor is selected from at least one of pseudoboehmite, α-alumina, θ-alumina, γ-alumina, and amorphous alumina; and/or
the molding pore-expanding agent is selected from at least one of polyethylene glycol cellulose, methyl cellulose, carboxymethyl cellulose, ethyl cellulose, hydroxyethyl cellulose and starch, and selected from at least one of polyvinyl alcohol, polyethylene glycol, polyacrylamide, polypropylene glycol and sesbania powder; and/or
the ratio of the mass of the molding pore-expanding agent to the total mass of the dry material is (0.5-25):100, preferably (5-13):100; and/or
the liquid is an acidic solution, and preferably, the acidic solution is selected from at least one of aqueous solutions of acetic acid, ethanedioic acid, diacetic acid, maleic acid, citric acid, oxalic acid, formic acid, tartaric acid, nitric acid, sulfuric acid, hydrochloric acid, phosphoric acid and hypochlorous acid, and/or the acidic solution has a concentration of 0.01-5 mol/L, preferably 0.2-4 mol/L.

**14.** Use of the catalyst according to any one of claims 1 to 3 or the catalyst prepared by the preparation method according to any one of claims 4 to 7 in a catalytic hydrogenation field, preferably in a catalytic hydrogenation of acetylene to prepare ethylene.

**15.** Use of the modified carrier according to any one of claims 8 to 10 or the modified carrier prepared by the preparation method according to any one of claims 11 to 13 in preparation of an eggshell catalyst, in particular an ultrathin eggshell-type highly dispersed catalyst.

25μm

Fig. 1

25μm

Fig. 2

**INTERNATIONAL SEARCH REPORT**

International application No. **PCT/CN2024/121420**

**A. CLASSIFICATION OF SUBJECT MATTER**

B01J23/44(2006.01)i; B01J35/53(2024.01)i; B01J32/00(2006.01)i; C07C5/05(2006.01)i; C07C5/09(2006.01)i; C07C11/04(2006.01)i; C07C11/06(2006.01)i

According to International Patent Classification (IPC) or to both national classification and IPC

**B. FIELDS SEARCHED**

Minimum documentation searched (classification system followed by classification symbols)

IPC:B01J23/-; B01J35/-; B01J32/-; C07C5/-; C07C11/-

Documentation searched other than minimum documentation to the extent that such documents are included in the fields searched

Electronic data base consulted during the international search (name of data base and, where practicable, search terms used)

CNTXT, CNKI, ENTXT, VEN: 壳, 核, 蛋, 厚度, 分散度, 加氢, 乙炔, shell, corn, thick+, disper+, hydrogenat+, acetylene

**C. DOCUMENTS CONSIDERED TO BE RELEVANT**

| Category* | Citation of document, with indication, where appropriate, of the relevant passages | Relevant to claim No. |
|---|---|---|
| X | 刘秀芳等 (LIU, Xiufang et al.). "蛋壳型 Pd/α-Al2O3 催化剂的制备及活性 (Preparation and Catalytic Activity of Egg-Shelled Catalyst Pd/a-Al2O3)" *催化学报 (Chinese Journal of Catalysis),* Vol. 30, No. 3, 31 March 2009 (2009-03-31), 213-217<br>text, sections 2.2 and 3 | 1-3 |
| Y | 刘秀芳等 (LIU, Xiufang et al.). "蛋壳型 Pd/α-Al2O3 催化剂的制备及活性 (Preparation and Catalytic Activity of Egg-Shelled Catalyst Pd/a-Al2O3)" *催化学报 (Chinese Journal of Catalysis),* Vol. 30, No. 3, 31 March 2009 (2009-03-31), 213-217<br>text, sections 2.2 and 3 | 4-7, 14 |
| Y | CN 107511178 A (CHINA PETROLEUM & CHEMICAL CORP. et al.) 26 December 2017 (2017-12-26)<br>description, paragraphs 11 and 16-24 | 4-7, 14 |
| X | CN 107511178 A (CHINA PETROLEUM & CHEMICAL CORP. et al.) 26 December 2017 (2017-12-26)<br>description, paragraphs 11 and 16-24 | 8-13, 15 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 December 2024** | **03 January 2025** |

| Name and mailing address of the ISA/CN | Authorized officer |
|---|---|
| **China National Intellectual Property Administration (ISA/CN)<br>China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/121420**

**C. DOCUMENTS CONSIDERED TO BE RELEVANT**

| Category* | Citation of document, with indication, where appropriate, of the relevant passages | Relevant to claim No. |
|---|---|---|
| X | CN 116854839 A (CHINA PETROLEUM & CHEMICAL CORP. et al.) 10 October 2023 (2023-10-10)<br>description, paragraphs 18-19 and 53-54 | 1-3 |
| A | CN 103447031 A (IFP ENERGIES NOUVELLES) 18 December 2013 (2013-12-18)<br>entire document | 1-15 |
| A | CN 102451722 A (CHINA PETROLEUM & CHEMICAL CORP. et al.) 16 May 2012 (2012-05-16)<br>entire document | 1-15 |
| A | US 2023148027 A1 (CHIYODA CORP.) 11 May 2023 (2023-05-11)<br>entire document | 1-15 |
| A | US 4519951 A (UOP INC.) 28 May 1985 (1985-05-28)<br>entire document | 1-15 |

Form PCT/ISA/210 (second sheet) (July 2022)

**INTERNATIONAL SEARCH REPORT**
**Information on patent family members**

International application No.
**PCT/CN2024/121420**

| Patent document cited in search report | | | Publication date (day/month/year) | Patent family member(s) | | | Publication date (day/month/year) |
|---|---|---|---|---|---|---|---|
| CN | 107511178 | A | 26 December 2017 | None | | | |
| CN | 116854839 | A | 10 October 2023 | None | | | |
| CN | 103447031 | A | 18 December 2013 | JP | 2013248612 | A | 12 December 2013 |
| | | | | JP | 6302627 | B2 | 28 March 2018 |
| | | | | DK | 2669005 | T3 | 02 January 2019 |
| | | | | EP | 2669005 | A1 | 04 December 2013 |
| | | | | EP | 2669005 | B1 | 29 August 2018 |
| | | | | US | 2017095797 | A1 | 06 April 2017 |
| | | | | US | 10099205 | B2 | 16 October 2018 |
| | | | | US | 2013303813 | A1 | 14 November 2013 |
| | | | | FR | 2991197 | A1 | 06 December 2013 |
| | | | | FR | 2991197 | B1 | 22 August 2014 |
| CN | 102451722 | A | 16 May 2012 | None | | | |
| US | 2023148027 | A1 | 11 May 2023 | TW | 202144076 | A | 01 December 2021 |
| | | | | EP | 4140578 | A1 | 01 March 2023 |
| | | | | EP | 4140578 | A4 | 24 January 2024 |
| | | | | WO | 2021214955 | A1 | 28 October 2021 |
| | | | | AU | 2020443246 | A1 | 05 January 2023 |
| | | | | JPWO | 2021214955 | A1 | 28 October 2021 |
| | | | | JP | 7466634 | B2 | 18 April 2024 |
| | | | | KR | 20220158289 | A | 30 November 2022 |
| | | | | CA | 3176199 | A1 | 28 October 2021 |
| US | 4519951 | A | 28 May 1985 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101730588 A
- CN 103769094 A
- CN 111229216 A


### Non-patent literature cited in the description


- **H. P. BOEHM**. Some Aspects of the Surface Chemistry of Carbon Blacks and Other Carbons. *Carbon*, 1994